# EUROPEAN PATENT APPLICATION

(11) **EP 1 133 990 A1**
(43) Date of publication of application: **19.09.2001**
(21) Application number: 01106631.3
(22) Date of filing: 16.03.2001
(51) Int. Cl.: A61K 35/50, A61P 35/00

(54) **Anti-tumor composition consisting of a fraction of gravid uterus or mammalian placenta**

(30) Priority: 17.03.2000 IT MI000561
(71) Applicant: Biava, Pier Mario, 20123 Milano (IT); Calvi, Ruggero, Monte Carlo (MC)
(72) Inventor: Biava, Pier Mario, 20123 Milano (IT); Calvi, Ruggero, Monte Carlo (MC)
(74) Representative: Gervasi, Gemma, Dr.

(57) **Abstract**

Anti-tumour composition obtained from gravid uterus or from mammalian placenta consisting of the fraction having molecular weight lower than 10,000 D of extracts of gravid uterus or placenta of not human mammals, taken during the period of embryonal differentiation.

## Description

### Prior art

Extracts from embryos or gravid uterus to be used as anti-tumour substances have been described in previous patents.

For example in the French patent A-2451193 the use of a homogenate consisting of a hydroalcoholic extract of cow, sheep or pig embryos for the treatment of tumours is described.

In the WO 98/11905 application a composition is described suitable for the treatment of tumours containing an extract of embryos or gravid uterus wherein the embryos are taken from oviparous or ovoviviparous animals and the gravid uterus is taken from mouse, rabbit, sheep or pig.

However said homogenates and said extracts show several drawbacks.

Above all their anti-tumour activity is very low.

Moreover said products are not susceptible of transformation in pharmaceutical forms suitable to give safety in the administration.

Finally said products give undesired side effects due to the presence of substances having allergic or toxic action, which are in the composition containing all the substances originally present in the embryo and in uterus.

### Summary of the invention

Now anti-tumour compositions obtained from gravid uterus or from placenta of not human mammals allowing to overcome the problems of the known technique have been found.

Said compositions include the fraction having molecular weight lower than 10,000 D of extracts of gravid uterus or placenta of not human mammals, taken during the period of embryonal differentiation, with the exclusion of the fraction having a molecular weight greater than 10,000 D.

Said compositions, beside allowing the overcome of the problems of the known technique, show a greater efficacy in the anti-tumour treatment. In particular, said compositions exert a decidedly higher anti-tumour activity with respect to the extract of the "in toto" uterus.

These and other characteristics of the compositions according to the present invention will be to a greater extent illustrated in the following detailed description.

### Detailed description of the invention

The present invention refers to anti-tumour compositions obtained from gravid uterus or from placenta of not human mammals, taken during the period of embryonal differentiation.

More particularly said compositions include the fraction having molecular weight lower than 10,000 D of extracts of gravid uterus or placenta of not human mammals, taken in the period of embryonal differentiation. The period of embryonal differentiation varies according to the species and it is known for each mammal.

For example for the mouse said period lies between the 4th and the 12th day of gestation.

For the pig said period lies between the 7th and the 30th day of gestation.

For the horse said period lies between the 10th and the 55th day of gestation even if the complete differentiation of some cutaneous appendages occurs after such period.

For the cow said period lies between the 7th and the 45th day of gestation.

The composition according to the present invention is prepared by the following procedure, which is substantially identical starting from the gravid uterus or the placenta.

The mammalian gravid uterus is taken in the described period, the embryo is discarded and the uterus portion in which the embryo was implanted is taken.

This portion is treated with a PBS (phosphate saline buffer) solution in an homogenizer.

The ratio between PBS solution and uterus portion lies between 5 and 20 ml of PBS per 100 mg of uterus portion and the homogenization treatment is repeated three times, each time for the duration of one minute.

The homogenized mixture is centrifuged at 2,000 revolutions per minute for 5 minutes.

The supernatant layer is taken which is filtered and in the filtrate the proteins are dosed for which a concentration ranging from 1 to 8 mg/ml is found.

The filtrate is diluted with PBS in the ratio from 1:1 to 1:8 by volume and the obtained solution is submitted to fractionation with molecular sieves separating the fraction of molecules having molecular weight lower than 10,000 D.

The examination of said protein fraction with preparative HPLC (High Performance Liquid Chromatography) pointed out the presence of 4 different molecules both in the fraction obtained from mouse uterus and in that one obtained from pig uterus, from horse uterus and from cow uterus.

More precisely 3 of said molecules have molecular weight ranging from 5,000 D to 10,000 D while the fourth molecule, which is that one resulting in higher concentration, has molecular weight lower than 5,000 D.

The fraction of extract from gravid uterus or placenta according to the present invention may be treated in various ways.

For example said fraction may be freeze-dried and kept at a temperature ranging from -4 °C to -20 °C to be subsequently used for therapeutic aim by dilution with bidistilled water, in injectable form.

The same fraction may be used for the preparation of solutions, pomades, pellets, tablets, capsules and suppositories, in mixture with pharmaceutically acceptable solvents or excipients, for pharmaceutical or dietetic-alimentary use.

Finally said fraction may be diluted with a glycero-alcoholic solution for the preparation of a mother solution of homeopathic preparations, for example according to the rules of the French or German Official Homeopathic Pharmacopoeia.

For illustrative aim of the invention the following Examples are reported.

### Example 1

### Preparation of a composition from mouse uterus at 6th day of gestation

A mouse gravid uterus is taken at the 6th day of gestation. The uterus is opened, the embryo is removed and the uterus portion in which the embryo was implanted is taken. As a whole 50 mg of uterine tissue are gathered.

Said uterine tissue is treated with 5 ml of PBS (phosphate saline buffer) in an homogenizer for 1 minute.

The homogenization operation is repeated 3 times.

The homogenized mixture is centrifuged at 2,000 revolutions per minute for 5 minutes and then the supernatant layer is taken which is subsequently filtered by a 0.45 micrometers pore filter.

On the filtrate the proteins which result to be 1 mg/ml are dosed.

The filtrate is submitted to fractionation by molecular sieves separating the fraction of molecules having molecular weight lower than 10,000 D.

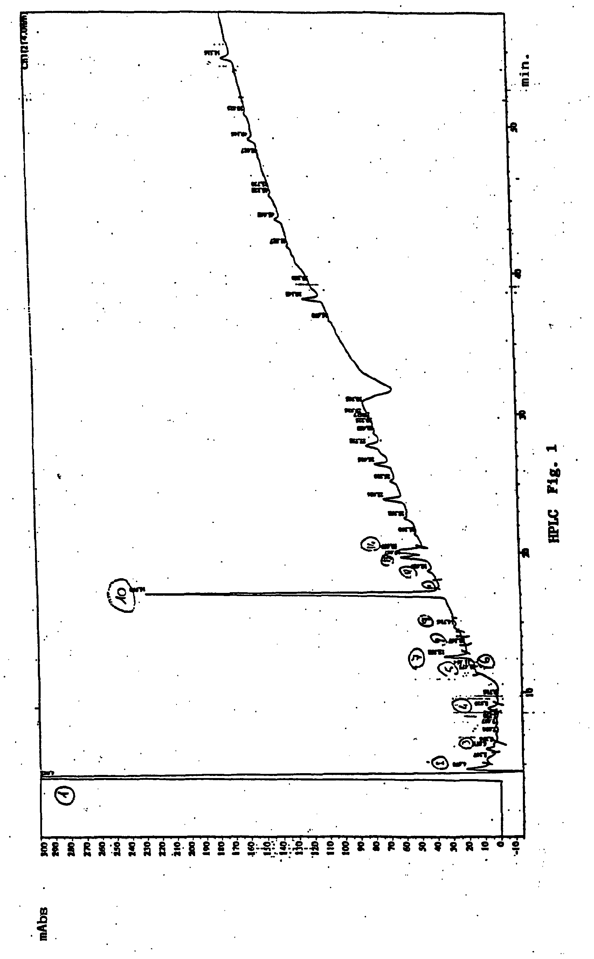

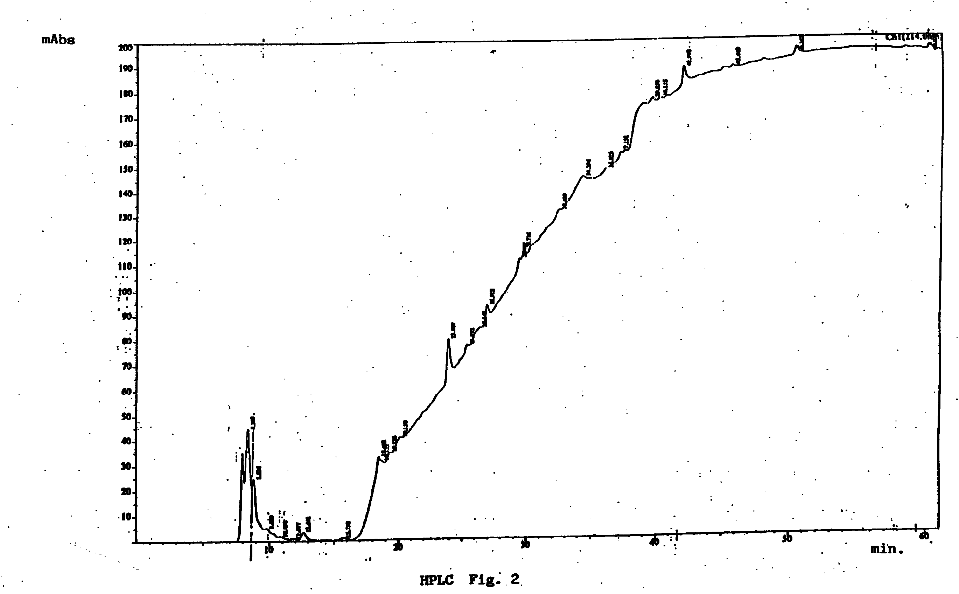

This fraction is freeze-dried and contains 25 µg of proteins and by HPLC analysis it results consisting of 4 different molecules of which, three have molecular weight higher than 5,000 D and the fourth one has a molecular weight lower than 5,000 D and a concentration greater than the other three. The HPLC plot is reported in the Figs. 1 and 2, wherein the peaks relating to the four molecules correspond respectively to peak 10 of Fig. 1 which one points out at about 17 minutes, and to peaks 1, 2 and 3 of the Fig. 2 which one points out starting from 7.5 minutes.

### Example 2

### Preparation of a composition from mouse uterus at the 11th day of gestation

A mouse gravid uterus is taken at the 11th day of gestation. As a whole 110 mg of uterine tissue are gathered.

This tissue is treated as described in the Example 1.

45 µg of proteins are obtained which by HPLC analysis result consisting of four different molecules as in the Example 1.

The HPLC plot is analogous to the Figs. 1 and 2.

### Example 3

### Preparation of a composition from pig uterus at the 24th day of gestation

A pig gravid uterus is taken on the 24th day of gestation.

The uterus is opened, the embryo is removed and the uterus portion in which the embryo was implanted is taken. As a whole 100 mg of uterine tissue are gathered.

Said tissue is placed in 10 ml of PBS and homogenized with homogenizer for 1 minute.

The homogenization operation is repeated 3 times.

The homogenized mixture is centrifuged at 2,000 revolutions per minute for 5 minutes and then the supernatant layer is taken which is subsequently filtered by a filter having 0.45 micrometer holes.

On the filtrate the proteins which result being in 2 mg/ml concentration are dosed. The filtrate is diluted by PBS solution in a ratio from 1:1 in volume and the obtained solution is submitted to fractionation by molecular sieves, separating the fraction of molecules having molecular weight lower than 10,000 D. Said fraction contains 50 micrograms of proteins.

This fraction at HPLC analysis turns out to contain 4 different molecules of which three having molecular weight greater than 5,000 D and one having molecular weight lower than 5,000 D, the latter being in greater concentration with respect to the other three.

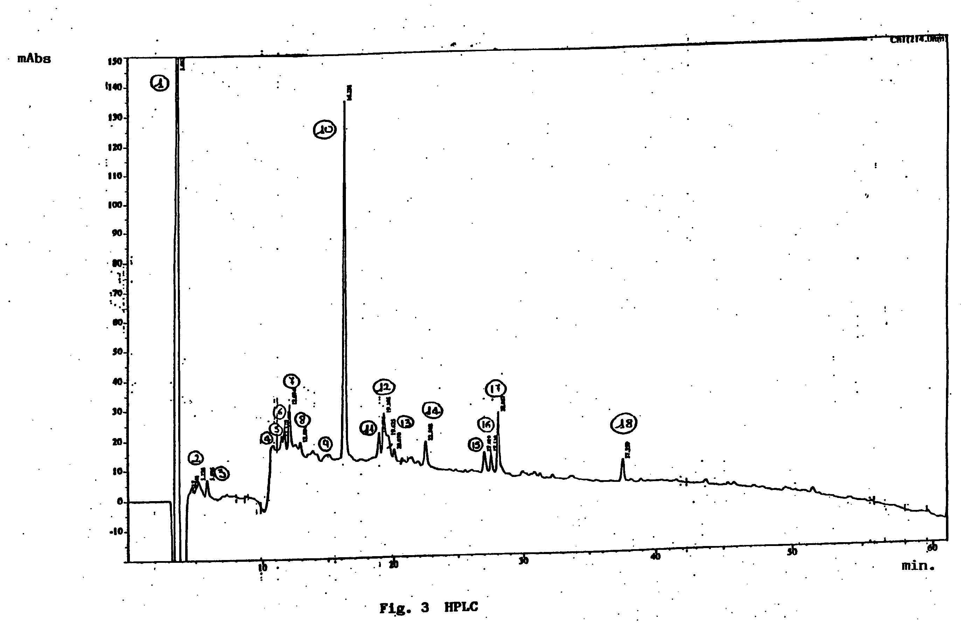

The HPLC plot is reported in Fig. 3, wherein the peaks relating to said molecules are 5, 6, 7 and 10 respectively.

Said 4 molecules may be also produced by synthesis processes.

### Biological experimentation

This experimentation has been carried out with the aim to verify the anti-tumour effect of the compositions according to the present invention.

A first experiment has been carried out using A172 Line human glioblastoma cells and the composition prepared according to the Example 1.

In ten 60 mm diameter Petri dishes 10,000 cells/plate have been plated in a culture medium consisting of 10% DMEM, 1% sodium pyruvate, 1% glutamine and 2% P/S.

Separately a solution of 50 µg of the freeze-dried product of the Example 1 in 100 µl of PBS has been prepared.

An amount of this solution corresponding to 10 µl has been added to each one of the 10 Petri dishes.

Five dishes have been maintained in culture for 24 hours and the remaining dishes have been maintained in culture for 48 hours.

At the end of these times the culture medium has been removed from each dish, the cells have been washed with 1 ml of PBS, treated with 1 ml of trypsin and resuspended in 1 ml of fresh culture medium in each dish. 500 µl of the suspension obtained from each dish have been diluted 1:1 with 0.2% Tripan Blue and we proceeded with the count of the cells in Bürker chambers (10 counts/sample, 15 µl of suspension/count).

For each dish 10 counts in Bürker chamber are carried out. The number of cells counted in the 10 counts is useful for obtaining the count average medium.

The control sample consists of 10,000 cells/dish in the culture medium consisting of 10% DMEM, 1% sodium pyruvate, 1% glutamine and 2% P/S, with no addition.

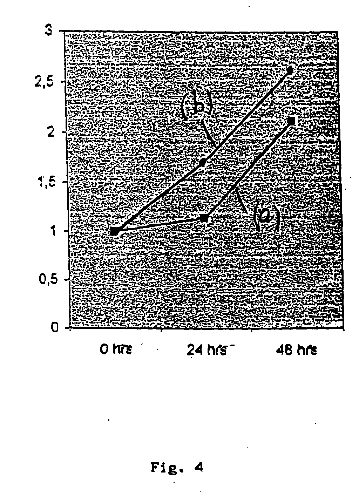

The results are reported in Figure 4 in which the (a) curve relates to the experiment with the composition of the Example 1 and the (b) curve relates to the control.

In Figure 4 and in the following Figures the cell count average value divided by 10 against time expressed in hours is represented. The above described experiment has been repeated with the composition obtained in the Example 2.

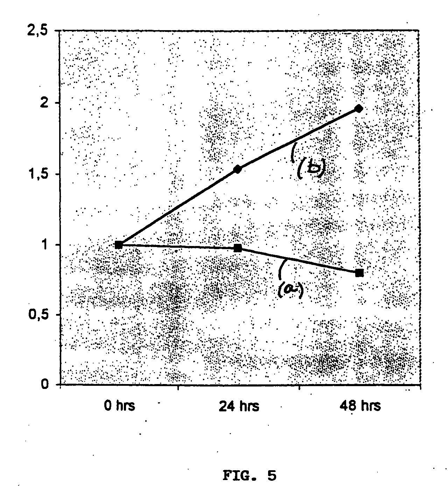

The results are reported in Fig. 5 in which the (a) curve relates to the experiment with the composition of the Example 2 and the (b) curve relates to the control.

The results confirm, with a clearly visible improvement, the results of the Example 1.

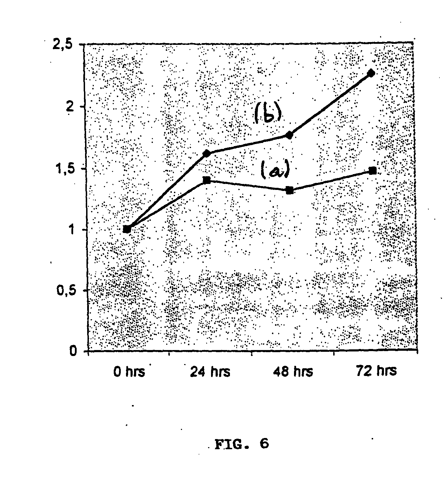

The above described experiment has been repeated with the composition of the Example 3 on ZR-75-1 Line mastocarcinoma human cells. The results are reported in Fig. 6, in which the (a) curve relates to the experiment with the composition of the Example 3 and the (b) curve relates to the control.

The biological experimentation has been completed by tests of treatment of A 172 Line human glioblastoma cells respectively with:
- extract of mouse "in toto" gravid uterus, taken at 6th day of gestation;
- fraction of gravid uterus greater than 10,000 D, taken from mouse at 6th day of gestation;
- fraction of gravid uterus greater than 30,000 D, taken from mouse at 6th day of gestation.

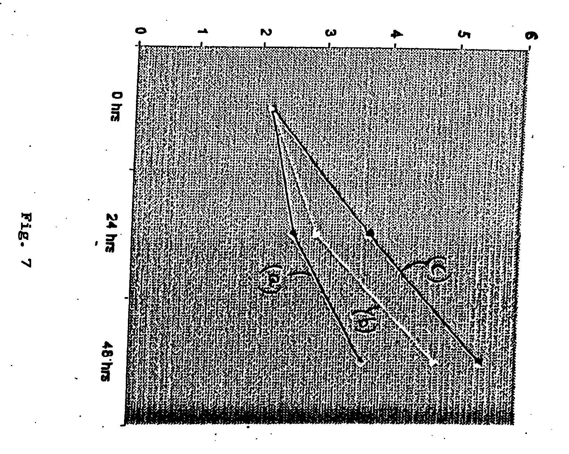

The results are reported in Figure 7 in which the (a) curve relates to the extract of the "in toto" gravid uterus, the (b) curve relates to the fraction greater than 10,000 D and the (c) curve relates to the fraction greater than 30,000 D.

From the above described experimentation turns out that the fraction of gravid uterus lower than 10,000 D reduces in a significant way the growth curve of human glioblastoma and of human mastocarcinoma in all the tests which have been carried out.

The reduction is clearly visible both in the experiment in which said fraction has been obtained from mouse uterus (Figs. 4 and 5) and in that one obtained from pig uterus (Fig. 6).

Moreover it must be pointed out that the extracts of mouse 6 days gravid uterus to which the 10,000 D fraction has been removed behave in a significantly different way with respect to the uterus total extract (Fig. 7). The treatment with the extracts to which the 10,000 D fraction has been removed does not reduce the growth curve in the same way as the extract of "in toto" uterus, but they reduce it in a significantly lower way.

The molecules contained in the fraction lower than 10,000 D have the effect to stop the growth of all the cells deviating from the normal behaviour. Said molecules do not stop only the growth of the tumoral cells (clearly abnormal) but also the growth of other cells which risk the embryo life as the activated lymphocytes. Therefore said fraction lower than 10,000 D is useful not only in the neoplastic diseases therapy, but also in the therapy of the autoimmune diseases and against the graft rejection.

The factors contained in such fraction may be therefore defined Life-Protecting Factors (LPF).

Of the four molecules present in said fraction, particularly active is the molecule of the fraction lower than 5,000 D, obtained by further fractionation of the fraction lower than 10,000 D with 5,000 D molecular sieves.

The fraction lower than 5,000 D has been experimented in the treatment of ZR-75-1 mastocarcinoma with the above described patterns.

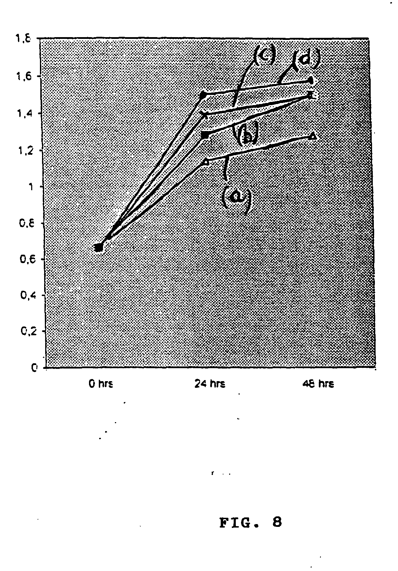

The results of the experiment have been reported in Fig. 8 in which the (a) curve relates to the treatment with the fraction lower than 5,000 D, the (b) curve relates to the treatment with the extract of the "in toto" uterus, the (c) curve relates to the treatment with the fraction ranging from 5,000 to 10,000 D and the (d) curve relates to the control.

## Claims

1. Anti-tumour compositions suitable for the treatment of the neoplastic diseases, of the autoimmune diseases and against implants rejection, obtained from gravid uterus or from mammalian placenta, **characterized in that** they include the fraction having molecular weight lower than 10,000 D of extracts of gravid uterus or placenta of not human mammals, taken during the period of embryonal differentiation.

2. Compositions as claimed in claim 1, **characterized in that** said gravid uterus or placenta are taken from the mouse in the period ranging from the 4th to the 12th day of gestation.

3. Compositions as claimed in claim 1, **characterized in that** said gravid uterus or placenta are taken from the pig in the period ranging from the 7th to the 30th day of gestation.

4. Compositions as claimed in claim 1, **characterized in that** said gravid uterus or placenta are taken from the horse in the period ranging from the 10th to the 55th day of gestation.

5. Compositions as claimed in claim 1, **characterized in that** said gravid uterus or placenta are taken from the cow in the period ranging from the 7th to the 45th day of gestation.

6. Compositions as claimed in claim 1, **characterized in that** said fraction includes 4 different protein molecules.

7. Compositions as claimed in claim 1, **characterized in that** said fraction contains a molecule having molecular weight lower than 5,000 D and three molecules having molecular weight ranging from 5,000 to 10,000 D.

8. Process for the preparation of anti-tumour compositions as defined in claim 1, wherein:
a) not human gravid uterus or placenta are taken during the period of embryonal differentiation;
b) the embryo is removed and the portion of uterus in which the embryo was implanted is taken;
c) the uterus portion coming from b) or the placenta coming from a) is treated with a PBS (phosphate saline buffer) solution in an homogenizer;
d) the homogenized mixture is centrifuged;
e) the supernatant layer obtained from the centrifugation is separated and filtered;
f) the filtrate of the e) step is diluted with PBS and submitted to fractionation with molecular sieves which separate the fraction of molecules having molecular weight lower than 10,000 D;
g) the fraction lower than 10,000 D of the e) step is freeze-dried.

9. Process as claimed in claim 8, **characterized in that** the ratio by weight between said PBS solution and said portion of uterus or said placenta of the step c) lies between 5 and 20 ml of PBS per 100 mg of uterus or placenta.

10. Process as claimed in claim 8, **characterized in that** said treatment in homogenizer of the step c) is repeated three times, each time for one minute duration.

11. Process as claimed in claim 8, **characterized in that** the centrifugation of the step d) is carried out at 2,000 revolutions per minute for 5 minutes.

12. Process as claimed in claim 8, **characterized in that** said dilution with PBS of the step f) is carried out with a 1:1 volume ratio.

13. Composition as claimed in claim 1 in freeze-dried form for therapeutic use.

14. Composition as claimed in claim 1 in mixture with pharmaceutically acceptable solvents or excipients for pharmaceutical or dietetic-alimentary use.

15. Composition as claimed in claim 1 in glycero-alcoholic solution for homeopathic use.
